# EUROPÄISCHE PATENTSCHRIFT

(11) **EP 2 532 637 B1**
(45) Veröffentlichungstag und Bekanntmachung des Hinweises auf die Patenterteilung: **28.10.2015**
(21) Anmeldenummer: 11004624.0
(22) Anmeldetag: 07.06.2011
(51) Int. Cl.: C07C 5/22, C07C 13/18

(54) **Verfahren zur Isomerisierung**
Isomerisation method
Procédé d'isomérisation

(43) Veröffentlichungstag der Anmeldung: 12.12.2012
(73) Patentinhaber: Merck Patent GmbH, 64293 Darmstadt (DE)
(72) Erfinder: Wächtler, Andreas, Dr., 64295 Darmstadt (DE); Stumpf, Hans-Bernd, 64646 Heppenheim (DE); Schäfer, Ralf, 63225 Langen (DE); Krattinger, Philipp, Dr., 64291 Darmstadt (DE)

(56) Entgegenhaltungen:
- DE-A1-102005 034 067
- DE-A1-102009 058 573
- DATABASE WPI Week 199725 Thomson Scientific, London, GB; AN 1997-276717 XP000002659224, & JP 9 100286 A (SHINETSU CHEM IND CO LTD) 15. April 1997 (1997-04-15)
- DATABASE WPI Week 200463 Thomson Scientific, London, GB; AN 2004-646561 XP000002659225, & JP 2004 256490 A (NAGASE CIBA KK) 16. September 2004 (2004-09-16)
- DATABASE WPI Week 199336 Thomson Scientific, London, GB; AN 1993-285371 XP000002659304, & JP 5 201881 A (CHISSO CORP) 10. August 1993 (1993-08-10)

## Beschreibung

Die vorliegende Erfindung betrifft ein Verfahren zur Herstellung von all-trans-4,4'-disubstituierten Bicyclohexanderivaten und deren Analoga, das einen Isomerisierungsschritt umfasst, der einen oder mehrere ciskonfigurierte Cyclohenxanringe unter Verwendung einer starken Säure in die trans-Konfiguration überführt.

Funktionale Chemikalien werden nicht nur durch ihre vorhandenen funktionellen Gruppen, sondern auch maßgeblich durch ihre Stereochemie, also ihrem räumlichen Aufbau, beeinflusst. Ein nicht unwesentlicher Teilbereich der Stereochemie ist einfachen nicht-aromatischen Ringen und der relativen Lage von Substituenten an diesen Ringen gewidmet.

Ein Grundproblem für die Stereochemie stellt die cis-trans-Isomerie von Substituenten an Cyclohexanen dar. Im Folgenden wird speziell auf die Isomerie von 1,4-substituierten Cyclohexanderivaten eingegangen. Obwohl für die Einstellung der Stereochemie von funktionalisierten Cyclohexanen (z. B. Cyclohexancarbonsäuren, Cyclohexylcarbaldehyden, Cyclohexanolen oder auch Phenylcyclohexanen) zahlreiche synthetische Methoden existieren, ist die Stereochemie von rein aliphatischen, nichtfunktionalisierten Cyclohexanen relativ schwierig zu steuern. Dass hierfür noch keine allgemein anwendbare Lösung existiert, ist insofern erstaunlich, als dass Verbindungen dieses Typs für technische Anwendungen schon seit langem bekannt sind (vgl. JP 59070624 A, 1984) und diese wegen des cis/trans-Problems aufwendig über die funktionalisierten Cyclohexanverbindungen hergestellt werden (vgl. CN 1962580 A).

Eine bekannte Reaktion ist die cis-trans-Isomerisierung von (4-Alkyl-cyclohexyl)-benzolen. Dabei lässt sich der phenylsubstituierte Cyclohexanring durch geeignete Maßnahmen dahingehend isomerisieren, dass aus einem cis-trans-Gemisch überwiegend das 1,4-trans-Isomer erhalten wird (JP 2004-256490 A). Im Gegensatz zum Gegenstand der vorliegenden Erfindung kann hierbei die Isomerisierung in Benzylstellung erfolgen. Die benzylische Position ist vergleichsweise leicht zur Isomerisierung zu bewegen. Daher verwenden herkömmliche Syntheserouten zur Herstellung von trans-Bicyclohexanen grundsätzlich Cyclohexylbenzol-Zwischenstufen, so dass wenigstens bereits einer der Cyclohexanringe in die trans-Konfiguration gebracht werden kann. An 4,4' dialkylierten Bicyclohexanen wurde bisher keine erfolgreiche Isomerisierung beschrieben.

Für Isomerisierungsverfahren bei Phenylcyclohexanen werden bislang starke Basen wie Kalium-tert-butylat oder bei funktionalen Cyclohexanderivaten beispielsweise Fluoridionen (DE 102005034067 A1) eingesetzt. Diese Isomerisierungsverfahren versagen jedoch bei unfunktionalisierten, d.h. rein aliphatischen 4,4'-Dialkylbicyclohexanen.

Die Druckschriften JP 09-100286 A und JP 05-201881 A offenbaren die cis-trans-Isomerisierung von 1,4-substituierten Cyclohexanen unter dem Einfluss von Lewis-Säuren.

Es wurde nun ein allgemein anwendbares Verfahren zur Herstellung von 1,4-disubstituierten Cyclohexanverbindungen mit all-trans Konfiguration der allgemeinen Formel I gefunden,

R¹-[A¹-(CH₂)ₙ-]ₘ-A²-R² I

worin
- R¹ und R²: unabhängig voneinander einen unsubstituierten, geradkettigen Alkylrest mit bis zu 9 C-Atomen oder -CH(CH₃)₂,
- m: 1 oder 2,
- n: jeweils unabhängig voneinander 0, 1, 2, 3 oder 4, bevorzugt 0, 2 oder 4 und
- A¹, A²: *trans*-1,4-Cyclohexyl,
bedeuten, das die Umsetzung von Verbindungen der Formel I entsprechenden 1,4-cis-Cyclohexanverbindungen in Gegenwart vorzugsweise katalytischer Mengen einer Carbokationen bildenden Verbindung (B) und in Gegenwart einer aus (B) Carbokationen generierenden und vorzugsweise auch stabilisierenden Protonensäure mit einem H₀-Wert < -11 umfasst. Die Säure ist vorzugsweise eine Supersäure, wobei die Verbindung (B) ausgewählt ist aus einem tertiären oder sekundären Alkylhalogenid oder -sulfonat, einem sekundären oder tertiären Alkohol, einem Alkylether oder Alkylsäureester eines tertiären oder sekundären Alkohols oder einem primären Halogenid, Sulfonat, Alkohol oder Ether mit Neopentylstruktur.

Als Edukte für das erfindungsgemäße Verfahren werden die entsprechenden Verbindungen der Formel I verwendet, bei denen ein Cyclohexanring oder mehrere Cyclohexanringe zumindest teilweise ciskonfiguriert vorliegen. In der Regel werden in das Verfahren cis/trans-Gemische eingesetzt, so wie sie aus den vorangehenden Synthesen entstehen. Der letzte Reaktionsschritt, der zur Ausgangsverbindung führt, ist meist eine Hydrierung, z.B. die Hydrierung von 4,4'-Dialkylbiphenyl, 1-Alkyl-4-(4-Alkyl-cyclohex-1-enyl)-benzol oder die Hydrierung von 1-Acyl-4-(4-alkylcyclohexyl)benzol zum cis/trans-Gemisch der entsprechenden 4,4'-Dialkylbicyclohexane.

Die Carbokationen bildende Verbindung (Katalysator) ist eine Verbindung, die unter den Reaktionsbedingungen geringe Mengen von carbokationischen Verbindungen erzeugt. Diese erfüllen die Rolle eines Katalysators.

Die Carbokationen bildende Verbindung wird im Folgenden als Katalysator bezeichnet. Der Katalysator wird bevorzugt in substöchiometrischen Mengen eingesetzt, d. h. in der Praxis genügen wenige mol% (0,05 bis 15 mol%) bezogen auf die zu isomerisierende Verbindung.

Abhängig von der Herstellung des Ausgangsmaterials (vide infra) ist in manchen Fällen eine Zugabe des Katalysators nicht nötig, weil bereits genügend Verunreinigungen enthalten sind, die die Rolle des Katalysators übernehmen. Dies kann insbesondere dann geschehen, wenn die Herstellung des Ausgangsmaterials über die Dehydratisierung eines Alkohols und anschließende Hydrierung oder über eine dehydratisierende Hydrierung verläuft und noch Spuren von Alkohol im zu isomerisierenden Gemisch verblieben sind. Bevorzugt wird der Katalysator jedoch eigens zugegeben.

Das Verfahren wird in einer bevorzugten Ausführungsform in Gegenwart eines sekundären oder tertiären Alkylhalogenids oder -sulfonats oder eines sekundären oder tertiären Alkohols als Katalysator durchgeführt. Bevorzugt wird als Katalysator ein tertiäres Halogenid, ein Halogenid an einem Brückenkopf eines polycyclischen Sytems, wie z.B. Adamantan, oder ein Neopentylhalogenid (primäres Halogenid) eingesetzt. Als Halogenide sind in diesem Zusammenhang Chlor, Brom oder Iod, insbesondere Chlor und Brom bevorzugt. Bei den Katalysatoren kann es sich auch um geminal mehrfach halogenierte Verbindungen, wie beispielsweise Haloform oder Tetrahalomethan, handeln. Anstelle eines Halogenids im Katalysator ist jeweils auch eine Hydroxylgruppe möglich. Beispiele von guten Carbokationenbildnern sind brückenkopfsubstituierte Adamantanverbindungen (bevorzugt Alkohol, Halogenid oder Alkylether) wie z. B. 1-Adamantanol oder 1-Halogenadamantan, außerdem Norborneol und Norbornylchlorid. Geeignete Katalysatoren sind außerdem die entsprechenden Ether der oben genannten Chloride oder Alkohole mit anderen einfachen Alkoholen wie Methanol, z. B. Methyl-tert-butylether. Besonders wirtschaftlich aufgrund der guten Verfügbarkeit und Wirksamkeit ist die Verwendung von tert-Butylchlorid (2-Chlor-2-methylpropan) bzw. tert-Butanol oder 1-Methycyclohexanol als Katalysator. Eine weitere Alternative sind tert.-Carbonsäuren/säurechloride , wie z.B. Pivaloylchlorid (2,2-Dimethylpropanoylchlorid), weil sich nach Abspaltung eines Chlorids unter Abspaltung von CO ein tertiäres Carbokation bilden kann.

Prinzipiell als Katalysator geeignet sind auch solche Alkene, die unter Addition von Protonen ein tertiäres Kation bilden können (z. B. 1-Methylcyclohexen).

Als Carbokationen bildende und stabilisierende Verbindungen eignen sich in erster Linie starke Säuren, bevorzugt Protonensäuren, beispielsweise solche mit einem H₀-Wert kleiner -11 (Supersäuren), bevorzugt perfluorierte Alkylsulfonsäuren, insbesondere Trifluormethansulfonsäure, Nonafluorbutansulfonsäure, und deren Derivate, wie Tris(trifluorsulfonyl)methan (CF₃SO₂)₂CH oder (CF₃SO₂)₂NH als Beispiele, wobei diese Beispiele nicht einschränkend gemeint sind. Wo nötig, kann die Stärke der Säure durch Kombination mit einer Lewis-Säure noch verstärkt werden. Dies kann besonders dann vorteilhaft sein, wenn man Alkene als Katalysatoren einsetzt. Viele weitere superacide Systeme in der flüssigen Phase sind aus der einschlägigen Literatur bekannt (vgl. Kapitel 2 in G.A. Olah in "Superacid Chemistry", 2. Ed., Wiley, 2009), wie Fluoroschwefelsäure und Gemische mit Lewissäuren wie SbF₅ etc ('magic acid'). Besonders bevorzugt sind allgemein nicht oxidierende Systeme, also solche die keine Schwefelsäure, Perschwefelsäure oder Schwefeltrioxid enthalten. Der Begriff Supersäure und der H₀-Wert werden in der zitierten Literatur definiert.

Der Katalysator wird ebenso wie im Allgemeinen auch die Säure in katalytischen Mengen eingesetzt, insbesondere in einer Menge, die noch geringer ist als die molare Menge der zugesetzten starken Säure. Bevorzugte Mengen des Katalysators sind 0,01 bis 2 mol% bezogen auf das Produkt oder 1 bis 90 mol%, bevorzugt 5 bis 25 % bezogen auf die Säure. Diese Mengenangaben sind nicht einschränkend zu sehen. Die Zugabe von zu großen oder gar stöchiometrischen Mengen an Katalysator und/oder Säure kann jedoch von Nachteil für das Verfahren sein, weil sich Nebenprodukte bilden.

Das Verfahren wird bevorzugt in einem chlorierten oder fluorierten Lösungsmittel, wie z.B. in Dichlormethan, mehrfach fluorierte Aromaten, z. B. Benzotrifluorid, oder chlorierten Fluorkohlenwasserstoffen durchgeführt. Geeignete Lösungsmittel sind aus der Chemie in Supersäuren bekannt (vgl. G. A. Olah, div. Publikationen).

Die Reaktionstemperatur im Verfahren beträgt bevorzugt von 20 bis -180°C, besonders bevorzugt von 0 °C bis -100 °C und ganz besonders bevorzugt von -30 °C bis -78 °C. Durch die niedrige Verfahrenstemperatur werden wenig Nebenprodukte erzeugt. Auch empfindliche Edukte können eingesetzt werden.

Das gewünschte Produkt wird in der Regel nach einer Reaktionszeit von 0,1 h bis 4 h erhalten.

Das erfindungsgemäße Verfahren zeichnet sich durch eine schonende Verfahrensweise aus, durch einen äußerst hohen trans-Anteil im Isomerisierungsprodukt und durch wenig Nebenprodukte. Die Abtrennung des zugesetzten Katalysators ist unproblematisch.

Die im erfindungsgemäßen Verfahren eingesetzten Cyclohexane mit einem zu isomerisierenden cis-Anteil werden nach üblichen Methoden hergestellt. Bewährte Ausgangsverbindungen sind 4-substituierte Cyclohexanone, beispielsweise 4-(4-Alkylcyclohexyl)cyclohexanone. Aus diesen Edukten bieten sich zweierlei Synthesewege zu den Cyclohexanen an: Durch Addition von Grignard- oder lithiierten-Verbindungen an die Carbonylgruppe mit anschließender Eliminierung und Hydrierung der so erhaltenen Alkene an gängigen Katalysatoren oder durch Wittig-Reaktion mit Alkylphosphoniumsalzen und anschließender Hydrierung. Aus den jeweiligen Hydrierungen resultieren in der Regel cis/trans-Gemische mit einem trans-Gehalt kleiner 85 %, meistens wesentlich darunter. Ein bevorzugtes Verfahren ist daher dadurch gekennzeichnet, dass die Konfiguration des Reaktionsprodukts an jedem 1,4-substituierten Cyclohexanring zu 94 % oder mehr der trans-Konfiguration entspricht. Dabei entspricht vorzugsweise die Konfiguration des Edukts an mindestens einem 1,4-substituierten Cyclohexanring zu 90 % oder weniger der trans-Konfiguration. Besonders bevorzugt entspricht die Konfiguration des Reaktionsprodukts an jedem 1,4-substituierten Cyclohexanring zu 97 % und ganz besonders bevorzugt zu 99% oder mehr der trans-Konfiguration. Das Produkt besitzt daher vorzugsweise zu 97 % oder mehr die all-trans-Konfiguration an den 1,4-Cyclohexanresten.

Eine weitere Synthese der zu isomerisierenden Cyclohexanverbindungen stellt die Hydrierung von entsprechenden Benzolverbindungen dar, beispielsweise von substituierten Cyclohexylbenzolen. Diese Verbindungsklasse lässt sich leicht aus chemischen Grundverbindungen erhalten. Geeignete Hydrierkatalysatoren und die Verfahrensparameter sind dem Fachmann vertraut.

Ein bevorzugtes Verfahren gemäß der Erfindung ist daher dadurch gekennzeichnet, dass es als einen weiteren Verfahrensschritt vor der erfindungsgemäßen Isomerisierung mit der Säure eine Hydrierung an einem Benzolring, einem Cyclohexenring oder einem Cyclohexadienring umfasst, wobei dieser Ring in einen Cyclohexanring überführt wird oder, dass es als einen Verfahrensschritt vor der Isomerisierung eine Hydrierung einer direkt an den Cyclohexanring gebundenen Alkylidengruppe umfasst. Vorzugsweise wird genau dieser Ring bei der Isomerisierung in die trans-konfiguration überführt, soweit er in der cis-Konfiguration aus der Hydrierung hervorgeht. Vorzugsweise geht die Hydrierung der Isomerisierung direkt voraus.

Das erfindungsgemäße Verfahren stellt eine effiziente Methode zur Isomerisierung des unerwünschten cis-Anteils der 1,4-Cyclohexanderivate dar. Das Verfahren kann zur direkten Erhöhung des trans-Anteils von cis/trans-Gemischen verwendet werden, selbst wenn der Anteil des trans-Isomeren bereits 85 % oder mehr beträgt. Der all-trans-Anteil nach der Isomerisierung beträgt vorzugsweise 94 % und mehr, besonders bevorzugt 97 % und mehr und insbesondere 99% und mehr. Ebenso kann das Verfahren zur Isomerisierung von Rückständen mit erhöhtem cis-Anteil aus der Anreicherung von trans-Isomeren, z.B. Mutterlaugen aus der Kristallisation, verwendet werden. So können auch nicht verwertbare Reste an cis-konfiguriertem Material durch Isomerisierung wiederverwertet werden.

In einer bevorzugten Ausführungform der Erfindung sind m = 1 und n = 0. (4,4'-disubstituierte Bicyclohexane), R¹ und R² sind bevorzugt n-Alkylreste mit 2 bis 7 C-Atomen oder ein Isopropylrest, wobei wiederum besonders bevorzugt die Gesamtsumme aller C-Atome in R₁ und R₂ acht nicht überschreitet. Einer der beiden Cyclohexanringe kann vor der Isomerisierung bereits die gewünschte trans-Konfiguration aufweisen, weil diese bei der Isomerisierung erhalten bleibt.

Für den Fall, dass die Formel I mehr als zwei Cyclohexanringe aufweist eignet sich das erfindungsgemäße Verfahren ebenfalls. Dabei kann auch bereits einer oder zwei der Cyclohexanringe die gewünschte trans-Konfiguration aufweisen, weil diese erhalten bleibt. Genauso ist es möglich, durch die hohe trans-Selektivität des Isomerisierungsverfahrens auch Isomerisierungen an drei Ringen gleichzeitig durchzuführen. Der Anteil der trans-trans- oder trans-trans-trans-Produkte (all-trans-Produkte) ist in der Regel hoch genug, um eine anschließende Abtrennung von cis-Derivaten überflüssig zu machen. Wird eine besonders hohe Reinheit der all-trans-Verbindungen angestrebt, so reicht in der Regel ein einziger Kristallisationsschritt aus, um Nebenprodukte, Katalysatorreste und die geringen cis-Anteile abzutrennen. Ohne eine effektives Isomerisierungsverfahren werden zur Abtrennung der cis-Isomere in der Regel mehrere Kristallisationsstufen benötigt und die Kristallisation des trans-Produkts wird durch das anwesende cis-Isomer und den smektogenen Charakter der Verbindungen erschwert.

Der Ausdruck "Alkyl" umfasst vorzugsweise unverzweigte oder verzweigte Alkylgruppen mit 1 bis 15 Kohlenstoffatomen, insbesondere die unverzweigten Gruppen Methyl, Ethyl, Propyl, Butyl, Pentyl, Hexyl und Heptyl. Gruppen mit 2 bis 10 Kohlenstoffatomen sind im allgemeinen bevorzugt. Als verzweigte Alkylgruppe ist die Isopropylgruppe bevorzugt.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfasst z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z.B. Extraktion), Adsorption an festen Trägern, Abdestillieren von Lösungsmitteln und/oder azeotropen Gemischen, selektive Destillation, Sublimation, Kristallisation, Cokristallisation oder durch Nanofiltration an Membranen.

In den Diagrammen bedeuten die Ringe 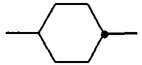 (mit Punkt) einen 1,4-trans-substituierten Cyclohexanring,
und 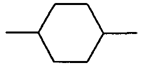 einen 1,4-substituierten Cyclohexanring mit gemischter
cis- und trans-Konfiguration oder überwiegend cis-Konfiguration.

Weitere bevorzugte Verfahrensvarianten lassen sich den Beispielen entnehmen, deren Details - auch verallgemeinert nach allgemeiner Fachkenntnis - repräsentativ für bevorzugte Ausführungsformen des erfindungsgemäßen Verfahren und seiner Produkte sind.

### Beispiele

### Allgemeines

Die Auswertung der entnommen Proben und die Bestimmung der Konfiguration des Endprodukts erfolgt mittels HPLC mit Acetonitril als Lösungsmittel an einer Purospher® STAR RP-18 Säule (Merck KGaA, Darmstadt). Die Zwischenproben werden mit Salzsäure hydrolysiert, mit Bicarbonat neutralisiert, extrahiert eingedampft und vermessen. eingedampft und vermessen.

### Beispiel 1: Isomerisierung mit Trifluormethansulfonsäure/1-Adamantanol

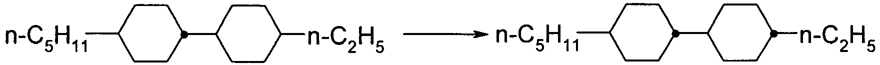

In 90 ml wasserfreiem Dichlormethan (zur Analyse) wird das Edukt (26,5 g; 100 mmol; cis/trans = 38 %/60 %) vorgelegt und auf -25 °C gekühlt. 0,660 ml Trifluormethansulfonsäure (etwa 7,5 mmol) werden zugegeben. Bei -25 °C werden 456 mg 1-Adamantanol (3,0 mmol) in 10 ml Dichlormethan während 10 min zugetropft. Der Ansatz wird bei -25 °C weiter gerührt, wobei alle 15 min eine Probe zur Bestimmung des Isomerisierungsgrades entnommen wird. Nach vollständiger Isomerisierung (0,2 - 3 h) wird der Ansatz aufgearbeitet.

Zur Aufarbeitung wird der Ansatz in eine Mischung aus 100 ml Salzsäure (25 %) und 50 g Eis eingerührt. Die organische Phase wird abgetrennt, mit 50 ml verdünnter NaHCO₃-Lösung und 100 ml Wasser gewaschen und am Rotationsverdampfer zum Rückstand eingeengt (22,9 g, 86,6 % d. Th.).

**Tabelle: Zeitlicher Verlauf des Isomerisierungsverfahrens**

| Reaktionszeit [min] | 15 | 30 | 60 | 270(*) |
|---|---|---|---|---|
| trans,trans-Anteil [%] | 89,68 | 93,93 | 96,42 | 99,64 |

| | | | | |
|---|---|---|---|---|
| (*) Isoliertes Endprodukt | | | | |

### Beispiel 2: Isomerisierung mit Trifluormethansulfonsäure/2-Chlor-2-methylpropan

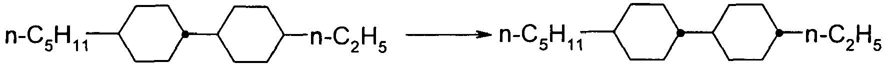

In 90 ml wasserfreiem Dichlormethan (zur Analyse) werden 26,45 g Edukt (100 mmol; cis/trans = 38 %/60 %) vorgelegt und auf -25°C gekühlt. Dazu gibt man 0,440 ml Trifluormethansulfonsäure und anschließend 0,22 ml 2-Chlor-2-methylpropan. Der Ansatz wird zwischen -20 °C und -25 °C 270 min gerührt, wobei nach jeweils 15, 30, 60, 120 und 180 min eine Probe zur Bestimmung des Isomerisierungsgrades entnommen wird. Es wird nach 270 Minuten wie in Beispiel 1 aufgearbeitet. Man erhält 22,9 g Produkt (86,6 % d. Th.).

**Tabelle: Zeitlicher Verlauf des Isomerisierungsverfahrens**

| Reaktionszeit [min] | 15 | 30 | 60 | 120 | 180 | 270(*) |
|---|---|---|---|---|---|---|
| all-trans-Anteil [%] | 78 | 86 | 95 | 97,5 | 98,57 | 98,8 |

| | | | | | | |
|---|---|---|---|---|---|---|
| (*) Isoliertes Endprodukt | | | | | | |

Weitere Kombinationen der Ausführungsformen und Varianten der Erfindung ergeben sich aus den Ansprüchen.

### Beispiel 3: Isomerisierung mit Trifluormethansulfonsäure/1-Chloradamantan

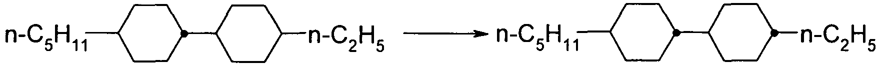

26,5 g Edukt (cis/trans-Gemisch: trans 60,4 %/cis 39,6 %) werden bei Raumtemperatur in 90 ml Dichlormethan vorgelegt, dann werden zu der Lösung 500 Mikroliter Trifluormethansulfonsäure gegeben. Unter Rühren wird auf -53° C abgekühlt, wobei eine rührbare Suspension entsteht. Zu dieser gibt man 341 mg 1-Chloradamantan gelöst in 10 ml Dichlormethan. Der Ansatz wird bei -50 °C weiter gerührt. Bereits nach 15 Minuten ist der HPLC-Gehalt einer Probe 98,5 % trans.

**Tabelle: Zeitlicher Verlauf des Isomerisierungsverfahrens**

| Reaktionszeit [min] | 15 | 60 |
|---|---|---|
| all-trans-Anteil [%] | 98,5 | 99,98 |

## Patentansprüche

1. Verfahren zur Herstellung von 1,4 -substituierten Cyclohexanverbindungen mit all-trans Konfiguration der allgemeinen Formel I
R¹-[A¹-(CH₂)ₙ-]ₘ-A²-R² I
worin
R¹ und R² unabhängig voneinander einen unsubstituierten, geradkettigen Alkylrest mit bis zu 9 C-Atomen oder -CH(CH₃)₂,
m 1 oder 2,
n jeweils unabhängig voneinander 0, 1, 2, 3 oder 4, und
A¹, A² *trans*-1,4-Cyclohexyl,
bedeuten,
das die Umsetzung von Verbindungen der Formel I entsprechenden 1,4-cis-Cyclohexanverbindungen in Gegenwart einer Carbokationen bildenden Verbindung (B) und in Gegenwart einer aus (B) Carbokationen generierenden Protonensäuremit einem H₀-Wert < -11 umfasst,
wobei die Verbindung (B) ausgewählt ist aus einem tertiären oder sekundären Allylhalogenid oder -sulfonat, einem sekundären oder tertiären Alkohol, einem Alkylether oder Alkylsäureester eines tertiären oder sekundären Alkohols oder einem primären Halogenid, Sulfonat, Alkohol oder Ether mit Neopentylstruktur.

2. Verfahren nach Anspruch 1, **dadurch gekennzeichnet, dass** es in Gegenwart einer Sulfonsäure oder eines Bis(sulfonyl)imids durchgeführt wird.

3. Verfahren nach Anspruch 1 oder 2, **dadurch gekennzeichnet, dass** es in Gegenwart von perfluorierter Alkylsulfonsäure durchgeführt wird.

4. Verfahren nach einem oder mehreren der Ansprüche 1 bis 3, **dadurch gekennzeichnet, dass** die Reaktion in Gegenwart eines oder mehrerer Zusätze in Form eines tertiären oder sekundären Alkylhalogenids, eines tertiären oder sekundären Alkohols oder an einem primären Halogenid oder Alkohol mit Neopentylstruktur durchgeführt wird.

5. Verfahren einem oder mehreren der Ansprüche 1 bis 4, **dadurch gekennzeichnet, dass** die Kationen bildende Verbindung ein brückenkopfsubstituiertes Adamantan ist.

6. Verfahren nach einem oder mehreren der Ansprüche 1 bis 5, **dadurch gekennzeichnet, dass** trans/trans-Bicyclohexan-Verbindungen der Formel IA 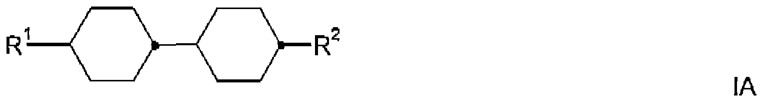 worin R¹ und R² wie in Anspruch 1 definiert sind,
hergestellt werden.

7. Verfahren nach einem oder mehreren der Ansprüche 1 bis 6, **dadurch gekennzeichnet, dass** die Reaktion bei Temperaturen kleiner oder gleich 20 °C durchgeführt wird.

8. Verfahren nach einem oder mehreren der Ansprüche 1 bis 7, **dadurch gekennzeichnet, dass** die Reaktion in einem chlorierten und/oder fluorierten Kohlenwasserstoff als Lösungsmittel durchgeführt wird.

9. Verfahren nach einem oder mehreren der Ansprüche 1 bis 8, **dadurch gekennzeichnet, dass** es als einen Verfahrensschritt vor der Isomerisierung hinführend zu den Verbindungen der Formel I eine Hydrierung an einem Benzolring, einem Cyclohexenring oder einem Cyclohexadienring umfasst, wobei diese Ringe in einen 1,4-substituierten Cyclohexanring überführt werden, oder, dass es als einen Verfahrensschritt vor der Isomerisierung eine Hydrierung einer direkt an den Cyclohexanring gebundenen Alkylidengruppe umfasst.

## Claims

1. Process for the preparation of 1,4-substituted cyclohexane compounds having an all-trans configuration of the general formula I
R¹-[A¹-(CH₂)ₙ-]ₘ-A²-R² I
in which
R¹ and R², independently of one another, denote an unsubstituted, straight-chain alkyl radical having up to 9 C atoms or -CH(CH₃)₂,
m denotes 1 or 2,
n in each case, independently of one another, denotes 0, 1, 2, 3 or 4, and
A¹, A² denote trans-1,4-cyclohexyl,
which comprises the reaction of 1,4-cis-cyclohexane compounds corresponding to compounds of the formula I in the presence of a carbocation-forming compound (B) and in the presence of a protonic acid having an H₀ value < -11 which generates carbocations from (B), where compound (B) is selected from a tertiary or secondary alkyl halide or sulfonate, a secondary or tertiary alcohol, an alkyl ether or alkyl acid ester of a tertiary or secondary alcohol, or a primary halide, sulfonate, alcohol or ether having a neopentyl structure.

2. Process according to Claim 1, **characterised in that** it is carried out in the presence of a sulfonic acid or bis(sulfonyl)imide.

3. Process according to Claim 1 or 2, **characterised in that** it is carried out in the presence of a perfluorinated alkylsulfonic acid.

4. Process according to one or more of Claims 1 to 3, **characterised in that** the reaction is carried out in the presence of one or more additives in the form of a tertiary or secondary alkyl halide, a tertiary or secondary alcohol, or on a primary halide or alcohol having a neopentyl structure.

5. Process according to one or more of Claims 1 to 4, **characterised in that** the cation-forming compound is a bridgehead-substituted adamantane.

6. Process according to one or more of Claims 1 to 5, **characterised in that** trans/trans-bicyclohexane compounds of the formula IA 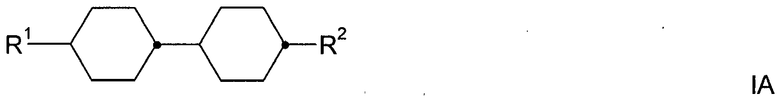 in which R¹ and R² are as defined in Claim 1,
are prepared.

7. Process according to one or more of Claims 1 to 6, **characterised in that** the reaction is carried out at temperatures of less than or equal to 20°C.

8. Process according to one or more of Claims 1 to 7, **characterised in that** the reaction is carried out in a chlorinated and/or fluorinated hydrocarbon as solvent.

9. Process according to one or more of Claims 1 to 8, **characterised in that** it comprises, as a process step before the isomerisation leading to the compounds of the formula I, a hydrogenation on a benzene ring, a cyclohexene ring or a cyclohexadiene ring, where these rings are converted into a 1,4-substituted cyclohexane ring, or **in that** it comprises, as a process step before the isomerisation, a hydrogenation on an alkylidene group bonded directly to the cyclohexane ring.

## Revendications

1. Procédé de préparation de composés de cyclohexane 1,4-substitué ayant une configuration tout-trans de formule générale I
R¹-[A¹-(CH₂)ₙ-]ₘ-A²-R² I
dans laquelle
R¹ et R², indépendamment l'un de l'autre, désignent un radical alkyle à chaîne linéaire non substitué, ayant jusqu'à 9 atomes de C ou -CH(CH₃)₂,
m désigne 1 ou 2,
n dans chaque cas, indépendamment l'un de l'autre, désigne 0, 1, 2, 3 ou 4, et
A¹, A² désignent *trans*-1,4-cyclohexyle,
comprenant la réaction de composés de 1,4-cis-cyclohexane correspondant aux composés de formule I en présence d'un composé formateur de carbocation (B) et en présence d'un acide protonique ayant une valeur H₀ < -11 qui génère des carbocations à partir de (B),
où le composé (B) est choisi parmi un halogénure ou sulfonate d'alkyle tertiaire ou secondaire, un alcool secondaire ou tertiaire, un alkyléther ou alkyl acide ester d'un alcool tertiaire ou secondaire, ou un halogénure, sulfonate, alcool ou éther primaire ayant une structure de néopentyle.

2. Procédé selon la revendication 1, **caractérisé en ce qu'**il est effectué en présence d'un acide sulfonique ou de bis(sulfonyl)imide.

3. Procédé selon la revendication 1 ou 2, **caractérisé en ce qu'**il est effectué en présence d'un acide alkylsulfonique perfluoré.

4. Procédé selon l'une ou plusieurs parmi les revendications 1 à 3, **caractérisé en ce que** la réaction est effectuée en présence d'un ou plusieurs additifs sous la forme d'un halogénure d'alkyle tertiaire ou secondaire, d'un alcool tertiaire ou secondaire, ou d'un halogénure ou alcool primaire ayant une structure de néopentyle.

5. Procédé selon l'une ou plusieurs parmi les revendications 1 à 4, **caractérisé en ce que** le composé formateur de cation est un adamantane substitué en tête de pont.

6. Procédé selon l'une ou plusieurs parmi les revendications 1 à 5, **caractérisé en ce que** les composés de trans/trans-bicyclohexane de formule IA 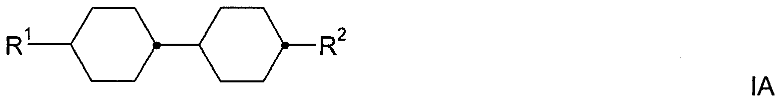 dans laquelle R¹ et R² sont tels que définis selon la revendication 1,
sont préparés.

7. Procédé selon l'une ou plusieurs parmi les revendications 1 à 6, **caractérisé en ce que** la réaction est effectuée à des températures inférieures ou égales à 20°C.

8. Procédé selon l'une ou plusieurs parmi les revendications 1 à 7, **caractérisé en ce que** la réaction est effectuée dans un hydrocarbure chloré et/ou fluoré comme solvant.

9. Procédé selon l'une ou plusieurs parmi les revendications 1 à 8, **caractérisé en ce qu'**il comprend, comme étape de procédé avant l'isomérisation conduisant aux composés de formule I, une hydrogénation sur un cycle benzène, un cycle cyclohexène ou un cycle cyclohexadiène, où ces cycles sont convertis en cycle cyclohexane 1,4-substitué, ou **en ce qu'**il comprend, comme étape de procédé avant l'isomérisation, une hydrogénation sur un groupement alkylidène lié directement au cycle cyclohexane.
